# EUROPEAN PATENT APPLICATION

(11) **EP 2 357 469 A1**
(43) Date of publication of application: **17.08.2011**
(21) Application number: 09830404.1
(22) Date of filing: 01.12.2009
(51) Int. Cl.: G01N 27/447, C07C 309/46

(54) **NOVEL CLEAR NATIVE ELECTROPHORESIS METHOD UTILIZING AROMATIC SULFONIC ACID COMPOUND**

(30) Priority: 02.12.2008 JP 2008307507
(71) Applicant: Japan Science and Technology Agency, Saitama 332-0012 (JP); Kyoto University, Sakyo-ku Kyoto-shi Kyoto 606-8501 (JP); Shizuoka Prefecture Public University Corporation, Shizuoka 422-8021 (JP)
(72) Inventor: HINO, Tomoya, Kyoto-shi Kyoto 606-8501 (JP); MURATA, Takeshi, Kyoto-shi Kyoto 606-8501 (JP); IWATA, So, Kyoto-shi Kyoto 606-8501 (JP); KAN, Toshiyuki, Shizuoka-shi Shizuoka 422-8526 (JP)
(74) Representative: HOFFMANN EITLE
(86) International application number: PCT/JP2009/070199
(87) International publication number: WO 2010/064638

(57) **Abstract**

This invention provides a reagent for protein electrophoresis, an electrophoresis gel or buffer composition containing the reagent, and a protein separation method and an electrophoresis kit using the reagent and the composition.

## Description

### Technical Field

The present invention mainly relates to an electrophoresis reagent, an electrophoresis composition, an electrophoresis kit, and a protein separation method.

### Background Art

Blue-Native electrophoresis has been frequently used as a process for examining the molecular weight or the association state of membrane proteins and supramolecular complexes while maintaining their natural states and enzymatic activities. This electrophoresis method, which was developed by Schagger et al. (Non-Patent Literature 1), uses a protein-binding property of Coomassie Brilliant Blue (hereinafter referred to as "CBB") G250, which has a negative charge, so the target proteins have a negative net charge. The negatively charged proteins are trapped in an acrylamide gel having a fine network structure, and a voltage is applied thereto to separate the proteins by molecular size. However, because the buffer used for this electrophoresis has a blue color derived from CBB G250, there is an inconvenience in adding the sample to the gel. Further, because the gel resulting from the electrophoresis has a blue color derived from CBB G250, it is not suitable for GFP fluorescence detection through electrophoresis of the GFP fusion protein.

Recently, Wittig et al. reported a Clear Native electrophoresis method using a deoxycholic acid (DOC), which is one kind of surfactant having a negative charge (see Non-Patent Literature 2). However, although Native electrophoresis using the transparent DOC is performed in a transparent state, there is a problem in that, for a certain kind of protein, multimeric proteins that would never exist in the natural state are generated.

### Citation List

### Non-patent Literature

[Non-patent Literature 1] Anal. Biochem. 1991 Dec 199 (2) 223-231
[Non-patent Literature 2] Mol. Cell Proteomics, 2007 Jul 6 (7) 1215-1225

### Disclosure of Invention

### Technical Problem

A main object of the present invention is to provide a reagent and a composition for protein electrophoresis that enable protein separation electrophoresis to be performed in a transparent state and in a state where the protein has a negative net charge while maintaining the higher-order structure or the complex structure of the protein; and to provide a protein separation method and an electrophoresis kit using the reagent and the composition.

### Technical Solution

The inventors of the present invention conducted extensive research to achieve the above object, and found that substantially the same electrophoresis pattern as in Blue-Native-electrophoresis can be obtained by using a specific compound that has little light absorption in the visible wavelength region. Based on this finding, the inventors conducted further extensive research and completed the present invention.

Specifically, the present invention relates to the following items.
Item 1. An electrophoresis reagent comprising at least one substantially colorless compound that binds to a protein in a neutral electrophoresis buffer and thereby forms a complex in which entire molecules are negatively charged.
Item 2. The electrophoresis reagent according to Item 1, comprising at least one arylsulfonate moiety.
Item 3. The electrophoresis reagent according to Item 1 or 2, comprising at least one substantially colorless compound that binds to a protein in a neutral electrophoresis buffer and thereby forms a complex in which entire molecules are negatively charged, the compound being represented by Formula (I) below: 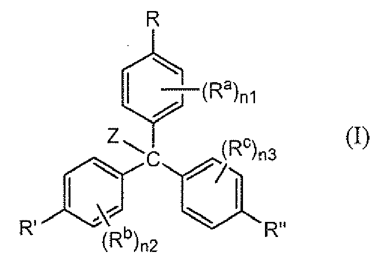 wherein, R represents a hydrogen atom, SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, carboxyl or NR¹R²; R' represents a hydrogen atom, SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, carboxyl or NR³R⁴; R" represents a hydrogen atom, SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, carboxyl or NR⁵R⁶; R¹, R², R³, R⁴, R⁵, and R⁶, which are the same or different, represent a hydrogen atom, alkyl, cycloalkyl, substituted or unsubstituted aryl or substituted or unsubstituted aralkyl; R^{a}, R^{b}, and R^{c}, which are the same or different, represent SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, amino, monoalkylamino, dialkylamino, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, acylamino or carboxyl; n¹, n² and n³, which are the same or different, represent an integer of 0 to 4; and Z represents a hydrogen atom, a halogen atom, OH, alkoxy, alkylcarbonyloxy, arylcarbonyloxy, aryloxy, SH, alkylthio, alkyl, amino (NH₂), monoalkylamino, dialkylamino or acylamino, provided that the compound of Formula (I) comprises 1, 2, 3, 4, 5 or 6 SO₃H or salts thereof.
Item 4. The electrophoresis reagent according to any one of Items 1 to 3, comprising at least one compound represented by General Formula (Ia) below, 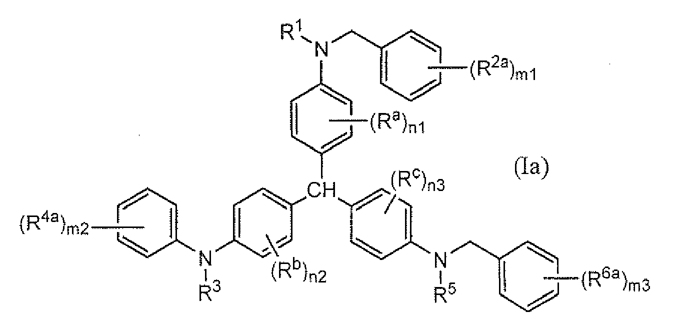 wherein, R¹, R³, and R⁵, which are the same or different, represent a hydrogen atom, alkyl, cycloalkyl, substituted or unsubstituted aryl or substituted or unsubstituted aralkyl; R^{a}, R^{b}, R^{c}, R^{2a}, R^{4a} and R^{6a}, which are the same or different, represent SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, amino, monoalkylamino, dialkylamino, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, acylamino or carboxyl; n¹, n² and n³, which are the same or different, represent an integer of 0 to 4; and m¹, m² and m³, which are the same or different, represent an integer of 0 to 5, provided that the compound of Formula (Ia) comprises 1, 2, 3, 4, 5 or 6 SO₃H or salts thereof, and 1 to 6 of R¹, R³, R⁵, 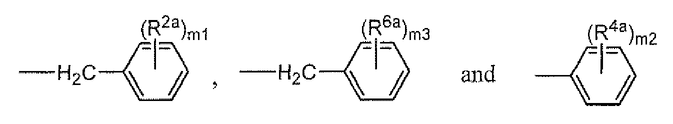 represent aryl or aralkyl having at least one SO₃H or salt(s) thereof.
Item 5. The electrophoresis reagent according to any one of Items 1 to 4, comprising at least one compound represented by General Formula (Ib) below, 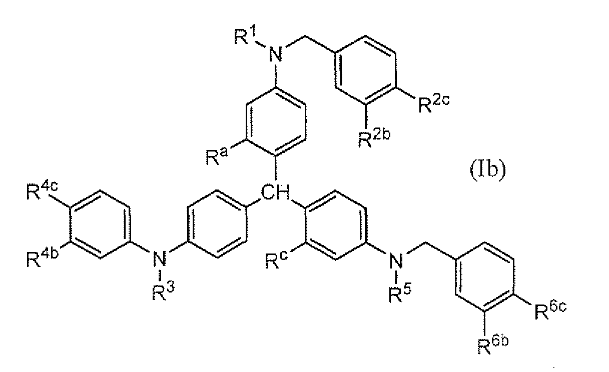 wherein, R¹, R³ and R⁵, which are the same or different, represent a hydrogen atom, C₁₋₄alkyl, substituted or unsubstituted phenyl or substituted or unsubstituted benzyl; R^{a} and R^{c}, which are the same or different, represent a hydrogen atom or methyl; one of R^{2b} and R^{2c} is a hydrogen atom while the other is SO₂NR⁹R¹⁰, SO₃H or a salt thereof; one of R^{4b} and R^{4c} is a hydrogen atom while the other is alkoxy; one of R^{6b} and R^{6c} is a hydrogen atom while the other is SO₂NR⁹R¹⁰, SO₃H or a salt thereof; R⁹ and R¹⁰ represent hydrogen or alkyl, provided that one or two of R^{2b}, R^{2c}, R^{6b} and R^{6c} represent SO₃H or salts thereof.
Item 6. The electrophoresis reagent according to any one of Items 1 to 5, comprising at least one compound represented by General Formula (IC) below, 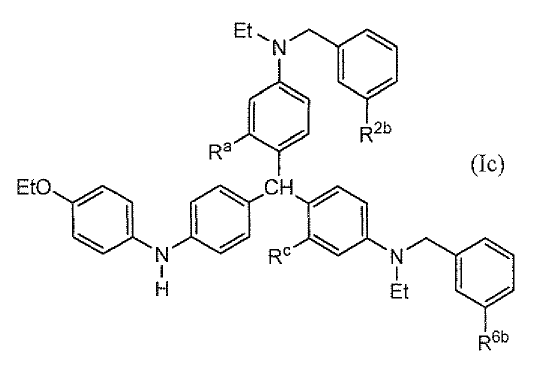 wherein, R^{a} and R^{c}, which are the same or different, represent a hydrogen atom or methyl; and R^{2b} and R^{6b} represent SO₃H or a salt thereof.
Item 7. An electrophoresis gel composition containing an electrophoresis gel and the electrophoresis reagent according to any one of Items 1 to 6.
Item 8. An electrophoresis buffer composition containing an electrophoresis buffer and the electrophoresis reagent according to any one of Items 1 to 6.
Item 9. A protein separation method comprising adding the electrophoresis reagent according to any one of Items 1 to 6 to a protein sample and/or an electrophoresis buffer; and performing Clear Native electrophoresis of a protein.
Item 10. A kit for protein electrophoresis, comprising at least one member selected from the group consisting of: the electrophoresis reagent according to any one of Items 1 to 6; the electrophoresis gel composition according to Item 7; and the electrophoresis buffer according to Item 8.
Item 11. A compound represented by one of Formulae (Ig) and (Ih) below, 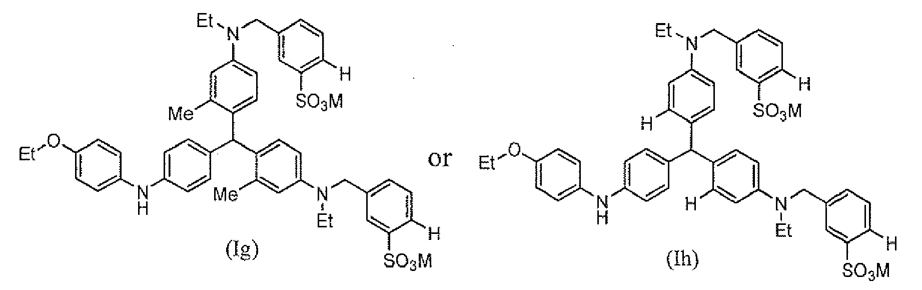 wherein M represents a hydrogen atom, a metal that forms a water-soluble salt, or N(R⁸)₄ (R⁸, which is the same or different, represents a hydrogen atom, alkyl, alkoxyalkyl, hydroxyalkyl, substituted or unsubstituted aryl, or substituted or unsubstituted aralkyl).

### Advantageous Effects of Invention

The reagent and the composition for electrophoresis according to the present invention enable electrophoresis in a transparent state, and also enable electrophoretic protein separation while maintaining a higher-order structure and complex structure with an overall negative charge.

Because the electrophoresis reagent of the present invention is substantially colorless, it is not necessary to perform a destaining process after the electrophoresis, thereby allowing the protein to be directly detected by way of silver-staining with significantly high sensitivity that is far superior to that of Blue-Native electrophoresis.

### Brief Description of Drawings

[FIG. 1]

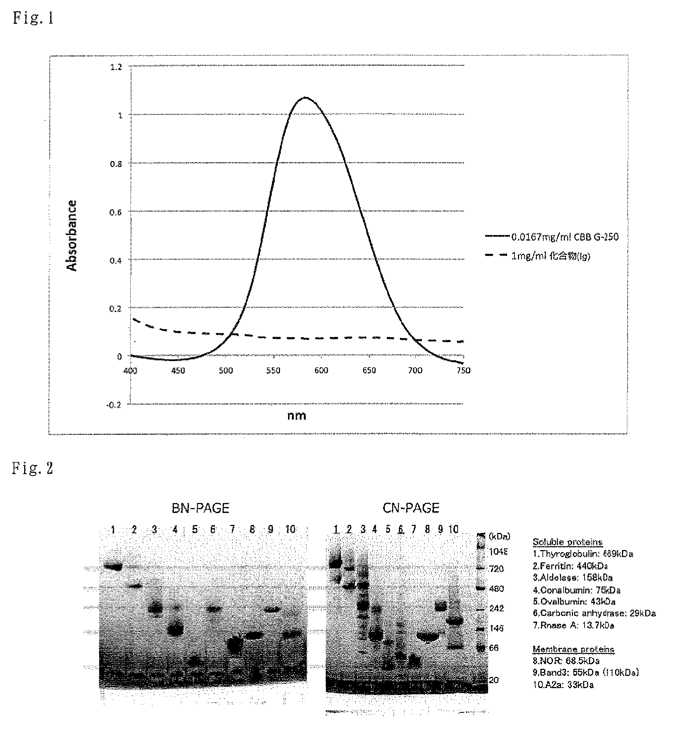

   FIG. 1 shows the measurement results of visible absorption spectra of Compound (Ig) and CBB G250. The vertical axis denotes absorbance, and the horizontal axis denotes wavelength (unit = nm). The solid line in FIG. 1 shows the measurement result for a 0.0167-mg/ml CBB G250 solution. The dashed line in FIG. 1 shows the measurement result for a 1-mg/ml Compound (Ig) solution.
[FIG. 2]
   FIG. 2 shows a comparison result of an electrophoresis pattern between various proteins obtained by performing Blue Native PAGE (hereinafter referred to as "BN-PAGE") and Clear Native PAGE ("CN-PAGE") for each protein. The left side of FIG. 2 shows patterns in the BN-PAGE, and the right side of FIG. 2 shows patterns in the CN-PAGE. The values in the horizontal axis of FIG. 2 show lane numbers, each of which denotes a type of protein, namely, 1: Thyroglobulin, 2: Ferritin, 3: Aldolase, 4: Conalbumin, 5: Ovalbumin, 6: Carbonic Anhydrase, 7: Ribonuclease A (RNase A), 8: Nitric Oxide Reduction enzyme (NOR), 9: Band 3, and 10: Adenosine receptor A2a (A2a). Nos. 1 to 7 are soluble proteins and Nos. 8 to 10 are membrane proteins.
[FIG. 3]

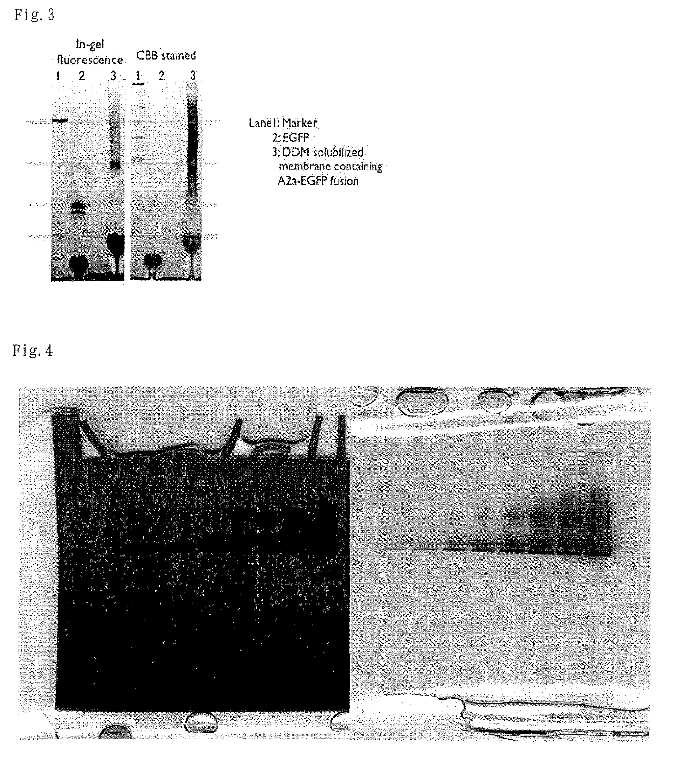

   FIG. 3 shows the results of fluorescence detection and CBB staining after CN-PAGE with respect to a fusion protein made of a protein sample and EGFP. The left side of FIG. 3 (In-gel fluorescence) shows the detection result through photofluorography for a gel after CN-PAGE, and the right side of FIG. 3 shows the detection result through CBB staining for a gel after CN-PAGE. The lanes 1, 2 and 3, respectively, show the results for a marker, EGFP, and an expressed membrane fraction of a fusion protein of a membrane protein and EGFP solubilized by dodecylmaltoside (DDM).
[FIG. 4]
   FIG. 4 shows the result of the detection of enzymatic activity in a gel after BN-PAGE or CN-PAGE using β-galactosidase. The left side of FIG. 4 shows the result of BN-PAGE, and the right side of FIG. 4 shows the result of CN-PAGE.
[FIG. 5]

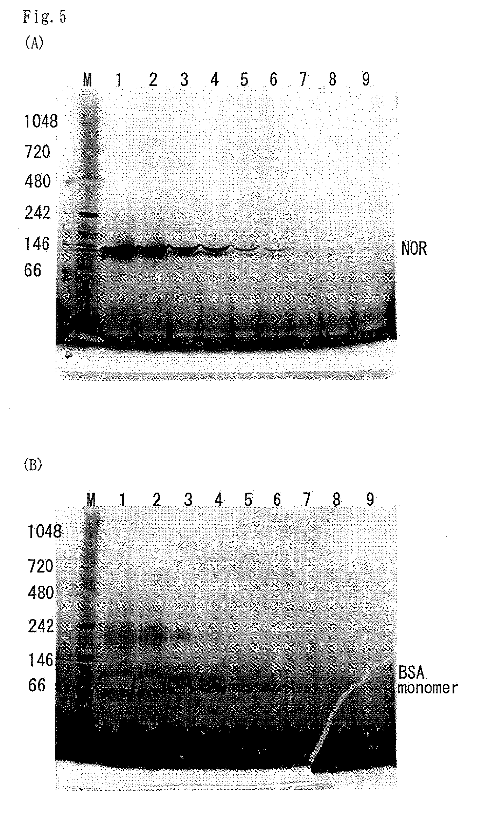

   FIG. 5 shows the results of silver staining after CN-PAGE using a membrane protein (NOR) and a water-soluble protein (BSA) as protein samples. FIG. 5 (A) shows the result of a NOR sample. FIG. 5 (B) shows the result of a BSA sample. The vertical axis in FIG. 5 denotes molecular weight (kDa), and the horizontal axis in FIG. 5 denotes the lane number given to the samples according to the electrophoresis amounts, namely, from left to right, M: marker, 1: 1,000 ng, 2: 500 ng, 3: 100 ng, 4: 50 ng, 5: 10 ng, 6: 5 ng, 7: 1 ng, 8: 0.5 ng, and 9: 0.1 ng.

### Description of Embodiments

The electrophoresis reagent used in the present invention contains a substantially colorless compound, which comprises an aryl moiety that binds to a hydrophobic moiety of a protein, and at least one SO₃H or a salt thereof that forms an anion in the neutral aqueous solution. By placing the compound in an aqueous solution so that the compound coexists with a protein, the entirety thereof forms an anionic complex.

In a preferable embodiment, the compound contained in the electrophoresis reagent comprises at least one aryl sulfonate moiety. The compound may further comprise at least one aryl amino moiety.

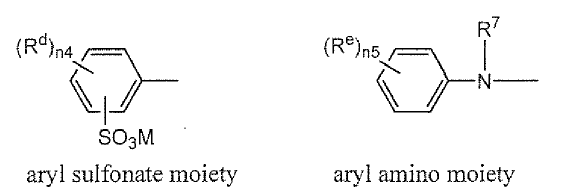

wherein, Rd and Re, which are the same or different, represent SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, amino, monoalkylamino, dialkylamino, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, acylamino or carboxyl.

R⁷ represents a hydrogen atom, alkyl, cycloalkyl, substituted or unsubstituted aryl, or substituted or unsubstituted aralkyl.

n4 represents an integer of 0 to 4. n5 represents an integer of 0 to 5.

M represents a hydrogen atom or metal that forms a water-soluble salt, particularly, an alkali metal such as Na, K, Li or Cs, or quaternary ammonium represented by N (R⁸)₄ (R⁸, which is the same or different, represents a hydrogen atom, alkyl, alkoxyalkyl, hydroxyalkyl, substituted or unsubstituted aryl or substituted or unsubstituted aralkyl).

Although the above are the cases in which the aryl in the aryl sulfonate moiety or in the arylamino moiety is a phenyl, the present invention, of course, includes aryl sulfonate moieties and arylamino moieties in which the aryl is a group other than phenyls, such as naphthyl, fluorenyl, anthryl, biphenylyl, tetrahydronaphthyl, chromanyl, 2,3-dihydro-1,4 dioxanaphthalenyl, indanyl, or phenanthryl.

The methylenedioxy, (-O-CH₂-O-) is formed by the integration of two adjacent substituents.

In a preferred embodiment, the compound is expressed by Formula (I).

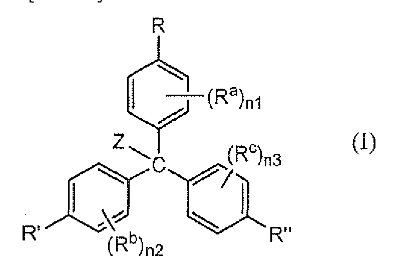

wherein R, R', R", R^{a}, R^{b}, R^{c}, n1, n2, n3 and Z are as defined above.

In a further preferred embodiment, the compound is expressed by Formula (I').

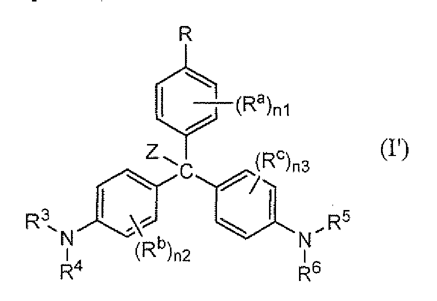

wherein, R, R³, R⁴, R⁵, R⁶ R^{a}, R^{b}, R^{c}, n1 n2, n3 and Z are as defined above.

In the present specification a "halogen atom" refers to fluorine, chlorine, bromine, or iodine. Among them, fluorine, chlorine, and bromine are preferable.

Examples of "alkyl" include linear, branched or cyclic C₁-₁₀ alkyl, preferably C₁₋₆ alkyl, more preferably C₁₋₄ alkyl, such as methyl, ethyl, n-propyl, isopropyl, n-butyl, isobutyl, tert-butyl, n-pentyl, isopentyl, hexyl, heptyl, octyl, nonyl, and decyl. Among them, methyl and ethyl are particularly preferable.

Examples of "cycloalkyl" include C₃₋₇ cycloalkyl such as cyclopropyl, cyclobutyl, cyclopentyl, cyclohexyl, and cycloheptyl.

Examples of "hydroxyalkyl" include C₁₋₄ alkyl having one OH such as hydroxymethyl, hydroxyethyl, hydroxy-n-propyl, hydroxyisopropyl, hydroxy-n-butyl, hydroxyisobutyl, and hydroxy-tert-butyl.

Examples of "alkoxyalkyl" include C₁₋₄ alkoxy C₁₋₄ alkyl such as methoxymethyl, methoxyethyl, ethoxymethyl, and ethoxyethyl.

The term "aryl" refers to a monocyclic or polycyclic group that has a 5- or 6-membered aromatic hydrocarbon ring or rings. Specific examples thereof include phenyl, naphthyl, fluorenyl, anthryl, biphenylyl, tetrahydronaphthyl, chromanyl, 2,3-dihydro-1,4-dioxanaphthalenyl, indanyl, fluorenyl and phenanthryl.

Examples of "aralkyl" include a linear, branched or cyclic C₁₋₄ alkyl substituted by the above aryl, such as benzyl, 1-phenylethyl, 2-phenylethyl, 1-prenylpropyl, 2-phenylpropyl, 3-phenylpropyl, and naphtylmethyl.

The term "alkenyl" refers to those having at least one double bond, such as linear, branched or cyclic C₂₋₁₀ alkenyl, preferably C₂₋₆ alkenyl, more preferably C₂₋₄ alkenyl, including vinyl, allyl, 1-propenyl, 2-methyl-2-propenyl, isopropenyl, 1-, 2-, or 3-butenyl, 2-, 3-, or 4-pentenyl, 2-methyl-2-butenyl, 3-methyl-2-butenyl, 5-hexenyl, 1-cyclopentenyl, and 1-cyclohexenyl, 3-methyl-3-butenyl.

The term "alkynyl" refers to those having at least one triple bond, such as linear, branched or cyclic C₂₋₁₀ alkynyl, preferably C₂₋₆ alkynyl, more preferably C₂₋₄ alkynyl, such as ethynyl, 1- or 2-propynyl, 1-, 2-, or 3-butynyl, and 1-methyl-2-propynyl.

Examples of "alkoxy" include linear, branched or cyclic C₂₋₆ alkoxy, such as methoxy, ethoxy, propoxy, isopropoxy, butoxy, isobutoxy, tert-butoxy, pentyloxy, isopentyloxy, and hexyloxy.

Examples of "monoalkylamino" include linear, branched or cyclic mono C₁₋₆ alkylamino, such as methylamino, ethylamino, n-propylamino, isopropylamino, n-butylamino, isobutylamino, tert-butylamino, n-pentylamino, isopentylamino and hexylamino.

Examples of "dialkylamino" include a linear, branched or cyclic di-C₁₋₆ alkylamino, such as dimethylamino, diethylamino, di-n-propylamino, diisopropylamino, di-n-butylamino, diisobutylamino, di-tert-butylamino, di-n-pentylamino, diisopentylamino, and dihexylamino.

Examples of "alkoxycarbonylamino" include linear, branched or cyclic C₁₋₆ alkoxycarbonyl amino, such as methoxycarbonylamino, ethoxycarbonylamino, propoxycarbonylamino, isopropoxycarbonylamino, butoxycarbonylamino, isobutoxycarbonylamino, tert-butoxycarbonylamino, pentyloxycarbonylamino, isopentyloxycarbonylamino and hexyloxycarbonylamino.

Examples of "monoalkylcarbamoyl" include C₁₋₆ alkylmonocarbamoyl, such as methylcarbamoyl, ethylcarbamoyl, n-propylcarbamoyl, isopropylcarbamoyl, n-butylcarbamoyl, isobutylcarbamoyl, tert-butylcarbamoyl, n-pentylcarbamoyl, isopentylcarbamoyl, and hexylcarbamoyl.

Examples of "dialkylcarbamoyl" include di-C₁₋₆ alkylcarbamoyl, such as dimethylcarbamoyl, diethylcarbamoyl, din-propylcarbamoyl, diisopropylcarbamoyl, di-n-butylcarbamoyl, diisobutylcarbamoyl, di-tert-butylcarbamoyl, di-n-pentylcarbamoyl, diisopentylcarbamoyl, and dihexylcarbamoyl.

Examples of "monoalkylsulfamoyl" include C₁₋₆ alkylmonosulfamoyl, such as methylsulfamoyl, ethylsulfamoyl, n-propylsulfamoyl, isopropylsulfamoyl, n-butylsulfamoyl, isobutylsulfamoyl, tert-butylsulfamoyl, n-pentylsulfamoyl, isopentylsulfamoyl, and hexylsulfamoyl.

Examples of "dialkylsulfamoyl" include di-C₁₋₆ alkylsulfamoyl, such as dimethylsulfamoyl, diethylsulfamoyl, din-propylsulfamoyl, diisopropylsulfamoyl, di-n-butylsulfamoyl, diisobutylsulfamoyl, di-tert-butylsulfamoyl, di-n-pentylsulfamoyl, diisopentylsulfamoyl, and dihexylsulfamoyl.

Examples of "alkylsulfonylamino" include a linear, branched or cyclic C₁₋₆ alkylsulfonylamino, such as methylsulfonylamino, ethylsulfonylamino, n-propylsulfonylamino, isopropylsulfonylamino, n-butylsulfonylamino, isobutylsulfonylamino, tert-butylsulfonylamino, n-pentylsulfonylamino, isopentylsulfonylamino, and hexylsulfonylamino.

Examples of "alkoxycarbonyl" include a linear, branched or cyclic C₁₋₆ alkoxycarbonyl, such as methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, isopentyloxycarbonyl, and hexyloxycarbonyl.

Examples of "monoarylamino" include phenylamino, naphthylamino, fluorenylamino, anthrylamino, biphenylamino, tetrahydronaphtylamino, chromanylamino, 2,3-dihydro-1,4-dioxanaphthalenylamino, indanylamino, fluorenylamino and phenanthrylamino.

Examples of "diarylamino" include diphenylamino, dinaphthylamino, difluorenylamino, dianthrylamino, dibiphenylamino, ditetrahydronaphtylamino, dichromanylamino, di (2, 3-dihydro-1,4-dioxanaphthalenyl) amino, diindanylamino, difluorenylamino and diphenanthrylamino.

Examples of "monoaralkylamino" include benzylamino, 1-phenylethylamino, 2-phenylethylamino, 1-phenylpropylamino, 2-phenylpropylamino, 3-phenylpropylamino, and naphthylmethylamino.

Examples of "diaralkylamino" include dibenzylamino, diphenethylamino, dipropylamino, and dinaphthylmethylamino.

Examples of "alkylcarbonyloxy" include a linear, branched or cyclic C₁₋₆ alkylcarbonyloxy, such as methylcarbonyloxy, ethylcarbonyloxy, n-propylcarbonyloxy, isopropylcarbonyloxy, n-butylcarbonyloxy, isobutylcarbonyloxy, tert-butylcarbonyloxy, n-pentylcarbonyloxy, isopentylcarbonyloxy, and hexylcarbonyloxy.

Examples of "arylcarbonyloxy" include phenylcarbonyloxy, naphthylcarbonyloxy, fluorenylcarbonyloxy, anthrylcarbonyloxy, biphenylylcarbonyloxy, tetrahydronaphthylcarbonyloxy, chromanylcarbonyloxy, 2,3-dihydro-1,4-dioxanaphthalenylcarbonyloxy, indanylcarbonyloxy, and phenanthrylcarbonyloxy.

Examples of "aryloxy" include phenyloxy, naphthyloxy, fluorenyloxy, anthryloxy, biphenylyloxy, tetrahydronaphthyloxy, chromanyloxy, 2,3-dihydro-1,4-dioxanaphthalenyloxy, indanyloxy, and phenanthryloxy.

Examples of "acylamino" include C₁₋₆ alkanoylamino, such as formylamino, acetylamino, propionylamino, or butyrylamino; and arylcarbonylamino such as benzoylamino.

Specific examples of alkylcarbonyloxy include C₁₋₆ alkylcarbonyloxy, such as methylcarbonyloxy, ethylcarbonyloxy, n-propylcarbonyloxy, isopropylcarbonyloxy, n-butylcarbonyloxy, isobutylcarbonyloxy, tert-butylcarbonyloxy, n-pentylcarbonyloxy, isopentylcarbonyloxy, and hexylcarbonyloxy.

Examples of arylcarbonyloxy include phenylcarbonyloxy, naphthylcarbonyloxy, fluorenylcarbonyloxy, anthrylcarbonyloxy, biphenylylcarbonyloxy, tetrahydronaphthylcarbonyloxy, chromanylcarbonyloxy, 2,3-dihydro-1,4-dioxanaphthalenylcarbonyloxy, indanylcarbonyloxy, and phenanthrylcarbonyloxy.

Specific examples of "alkylthio" include a linear, branched or cyclic C₁₋₆ alkylthio, such as methylthio, ethylthio, n-propylthio, isopropylthio, n-butylthio, isobutylthio, tert-butylthio, n-pentylthio, isopentylthio, or hexylthio.

Examples of the substituents of substituted or unsubstituted aryl or substituted or unsubstituted aralkyl include halogen atom, alkyl, alkoxy, SO₃H or salts thereof, hydroxy, trifluoro methoxy, trifluoro ethoxy, cyano, nitro, amino, monoalkylamino, dialkylamino, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, acylamino and carboxyl. The number of substituents is generally 1 to 4, preferably 1 to 3, more preferably 1 or 2.

n1, n2, n3, m1, m2 and m3, which are the same or different, are integers of 0 to 4, preferably 0 to 3, and more preferably 0, 1 or 2.

1 to 6, preferably 1 to 4, more preferably 1 to 3, and particularly preferably 1 to 2 of R¹, R², R³, R⁴, R⁵, and R⁶ represent an aryl or aralkyl having at least SO₃H or a salt thereof as a substituent.

The salt of SO₃H is not particularly limited insofar as it is a water-soluble salt of SO₃H. Examples thereof include a sulfonic acid salt represented by SO₃M (M is a hydrogen atom or metal that forms a water-soluble salt, particularly an alkali metal, such as Na, K, Li, or Cs, or an ammonium represented by N(R⁸)₄ (R⁸, which is the same or different, represents a hydrogen atom, alkyl, alkoxyalkyl, hydroxy alkyl, substituted or unsubstituted aryl, or substituted or unsubstituted aralkyl)).

The compound of the present invention comprises at least one SO₃H (sulfo group) or a salt thereof as a substituent. Thereby, the compound of the present invention binds to a protein through the sulfo group, and electrophoresis is carried out in a state with an overall negative charge. Although the color components used for conventional Blue-Native electrophoresis, such as CBB G250, CBB R250, etc., comprise two sulfo groups, they have an overall monovalent negative charge because they have an ammonium cation in the molecules. The compound of the present invention has at least a monovalent negative charge in the entire molecule. If the compound of the present invention does not have a cation, such as an ammonium ion, in the molecule, the compound may comprise two or more SO₃H or salts thereof (1 to 6); however, one SO₃H or a salt thereof suffices. Compounds (Ig) and (Ih) of the present invention produced by reducing CBB G250 and CBB R250 have two SO₃H or salts thereof, and are present as a bivalent anion in an aqueous solution. As such, when the compound of the present invention is present as a bivalent or polyvalent anion, disassociation of a subunit or partial damage of the tertiary structure may occur due to the strong negative charge depending on the type of protein or the conditions of electrophoresis. Therefore, the protein structure may be retained by replacing at least one sulfo group with a functional group having no charge, such as hydrogen, a hydroxy group (OH), sulfamoyl (-SO₂NH₂), mono- or di-alkylsulfamoyl, so that the compound has a monovalent negative charge, thereby easing the effect on the protein. Preferable examples of the compounds of the present invention include (Ii), (Ij), (Ik) and (Il), in addition to (Ig) and (Ih).

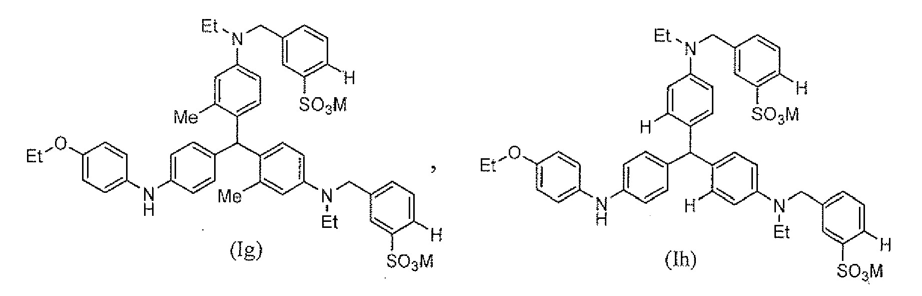

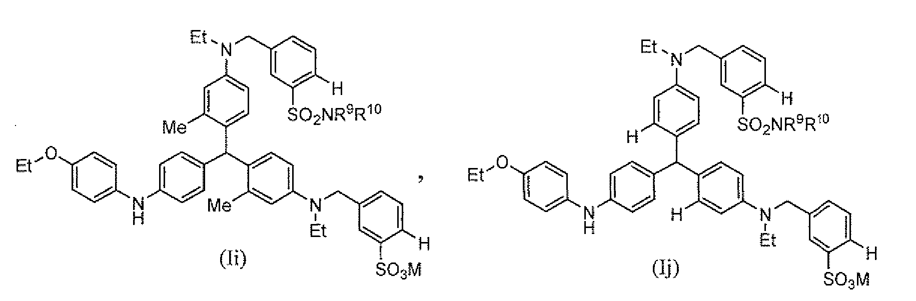

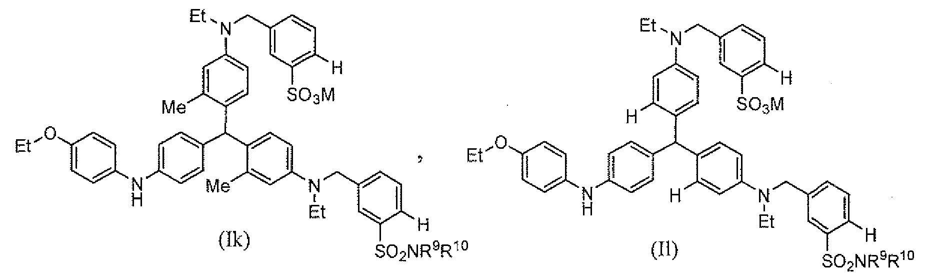

wherein, R⁹ and R¹⁰, which are the same or different, represent hydrogen or alkyl.

When reducing a dye compound having multiple sulfo groups such as CBB to obtain the colorless compound of the present invention, it is possible to obtain a colorless compound having the same charge as that of the dye compound while maintaining the same protein-binding property or water solubility as that of the dye compound, for example, by substituting one of the sulfo groups with another group such as a substituted or unsubstituted sulfone amide which does not have a charge but has water solubility or polarity.

The compound represented by General Formula (1) can be synthesized using a general chemical synthetic procedure. The following Scheme 1 shows a specific method.

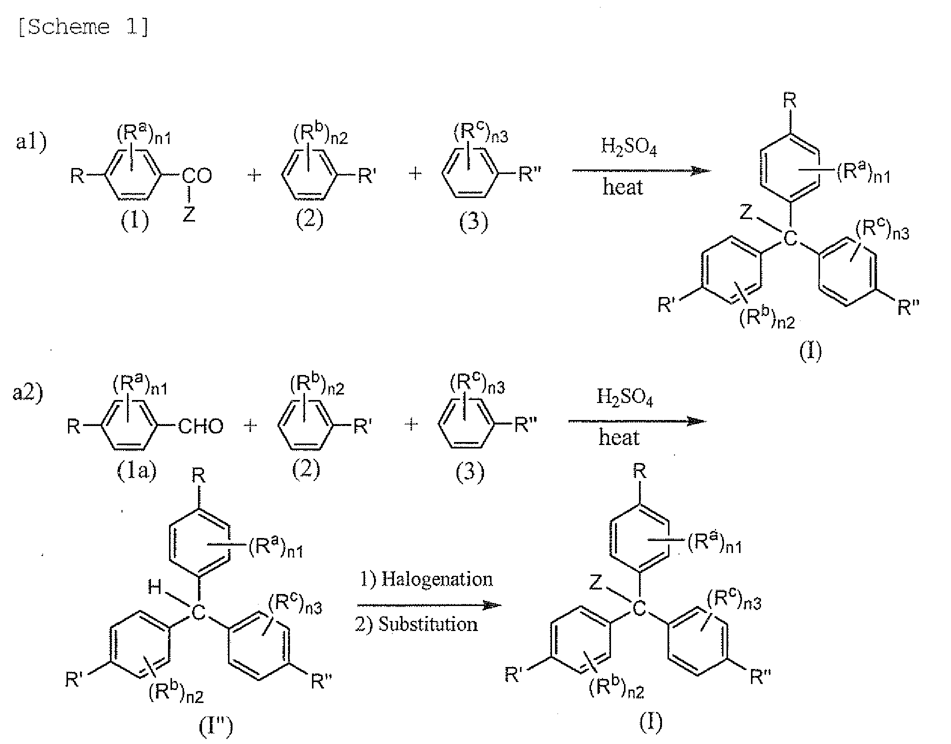

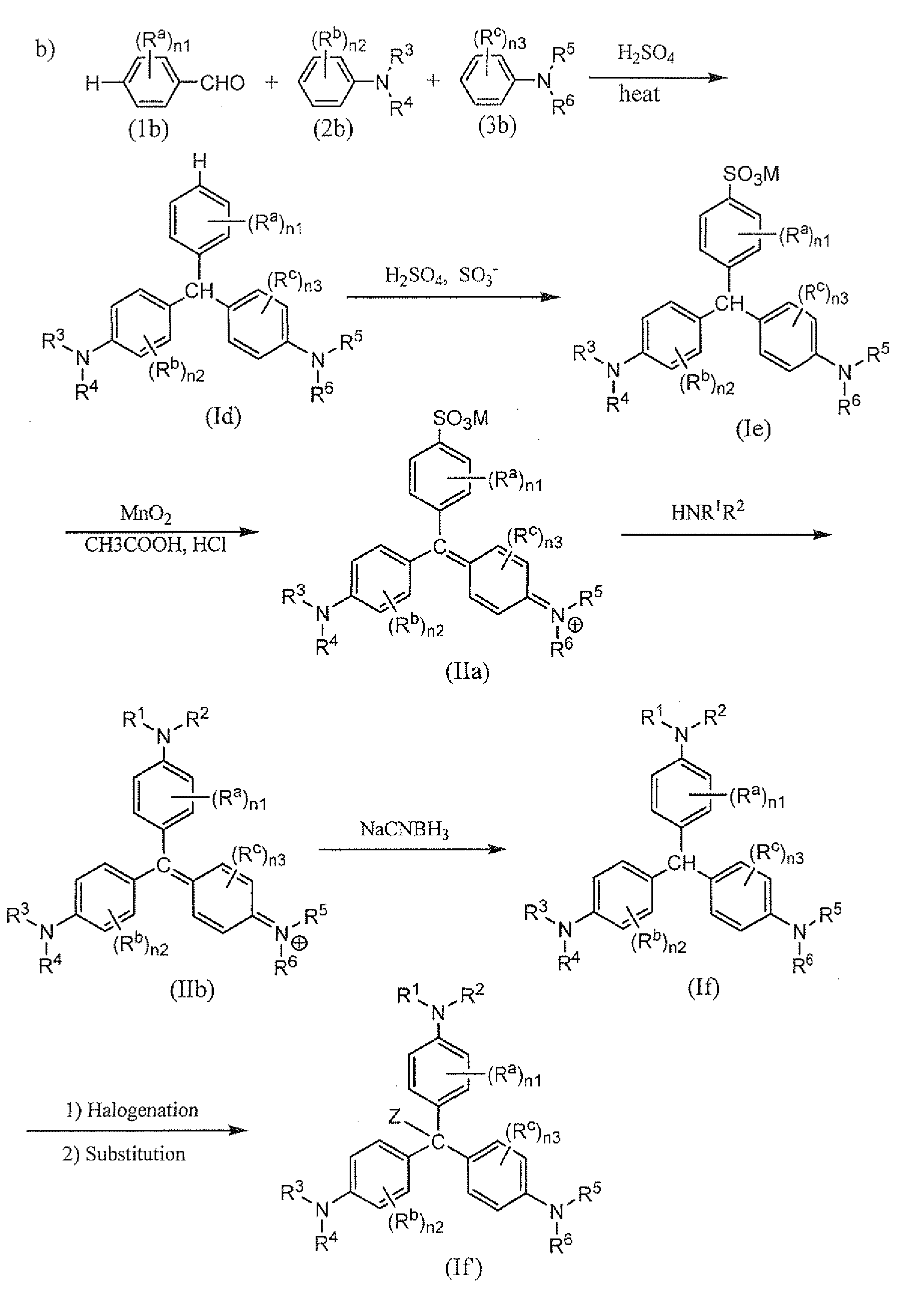

wherein R, R', R", R¹, R², R³, R⁴, R⁵, R⁶, R^{a}, R^{b}, R^{c}, n1, n2, n3 and Z are as defined above.

As shown in Scheme 1 (a1), Compound (I) of the present invention can be synthesized in a single step of reacting aldehyde (1), Compound (2), and Compound (3).

The reaction advantageously proceeds by using about 1 mol each of Compound (2) and Compound (3) with 1 mol of Compound (1), and a catalytic-to-excessive quantity of sulfuric acid, and reacting them for 5 to 96 hours at 60 to 150 °C in the presence of a solvent. Compound (2) and Compound (3) are preferably the same. When Compound (2) and Compound (3) are different, Compound (1) is produced either by reacting two molecules of Compound (2), two molecules of Compound (3), or one molecule each of Compound (2) and Compound (3), with aldehyde (1). By separating these compounds using a general separation method, such as column chromatography, recrystallization, solvent extraction, or preparative HPLC, each reaction produces the compound of Formula (I). Examples of solvents include alcohols, such as methanol, ethanol, or propanol, and ethers, such as tetrahydrofuran, dioxane, 1,2-dimethoxyethane, or diglyme.

As shown in Scheme 1 (a2), the Compound (I") where Z = H of the present invention may be synthesized by a single step of reacting aldehyde (la) with Compound (2) and Compound (3). Moreover, by introducing Z other than a hydrogen atom by subjecting this compound to (1) halogenation and, as required, (2) substitution of a halogen atom with another functional group, the compound of Formula (I) may be obtained. Halogenation is performed by reacting with halogen molecules, such as F₂, Cl₂, Br₂, or I₂, or a halogenation agent, such as N-bromo succinimide, or N-chloro succinimide. By reacting a compound where Z = halogen with an alkali metal hydroxide such as sodium hydroxide, or compounds such as (alkyl)-OH, (alkyl)-COOH, (aryl)-COOH, (aryl)-OH, (alkyl)-SH, ammonia, (alkyl)-NH_{2,} or (alkyl)₂-NH, in the presence of bases such as triethylamine or pyridine as required, it is possible to obtain compounds where Z = OH, alkoxy, alkylcarbonyloxy, arylcarbonyloxy, aryloxy, SH, alkylthio, alkyl, amino (NH₂), monoalkylamino, or dialkylamino. By reacting a compound where Z = an amino group with an acid anhydride such as acyl chloride, it is possible to obtain a compound where Z = acylamino.

As shown in Scheme I(b), Compound (Id) of the present invention may also be synthesized by a single step of reacting aldehyde (Ib), Compound (2b), and Compound (3b) in the presence of a solvent and a sulfuric acid. The reaction more advantageously proceeds by reacting about 1 mol each of Compound (2b) and Compound (3b) per mol of aldehyde (1b) with a catalytic-to-excessive quantity of sulfuric acid in the presence of a solvent for 5 to 96 hours at 60 to 150°C.

Further, by reacting Compound (Id) for 1 to 48 hours at a temperature ranging from room temperature to about 40°C in the presence of a sulfonating agent, such as concentrated sulfuric acid, or fuming sulfuric acid, it is possible to obtain sulfonic acid Compound (Ie) . Then, by oxidizing Compound (Ie) with MnO₂ in the presence of acetic acid and HCl, it is possible to obtain Compound (IIa), which is further reacted with HNR¹R² to obtain the compound of Formula (IIb). By further reducing Compound (IIb) with a reducing agent such as NaCNBH₃, it is possible to obtain Compound (If).

Compound (If) may be converted into Compound (If') by introducing a Z-group in the same manner as described above. By reacting Compound (Ie) with an equimolar-to-excessive amount of MnO₂, using acetic acid and HCl as solvents, for 1 to 24 hours at approximately room temperature, it is possible to obtain Compound (IIa). By reacting 1 mol of Compound (IIa) with 1 mol to an excessive amount of HNR¹R² in a solvent such as methanol or a like alcohol, methylene chloride or a like chlorinated hydrocarbon, etc., it is possible to obtain Compound (IIb). Further, by reducing 1 mol of Compound (IIb) by an equimolar-to-excessive amount of a reducing agent such as NaCNBH₃ in methanol or a like alcohol, it is possible to obtain Compound (If).

Alternatively, by reducing Compound (IIa), (IIb), or an acid dye having the following partial structure (i.e., a dye having 1 to 6 sulfo groups or salts thereof), such as CBB dye, as a raw material, which is derivatized as required, with a reducing agent such as NaCNBH₃, it is possible to obtain the compound of the present invention that has the following substantially colorless partial structure.

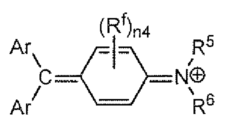

wherein, Ar, which is the same or different, represents substituted or unsubstituted aryl. R⁵ and R⁶ are as defined above. R^{f}, which is the same or different, represents SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, amino, monoalkylamino, dialkylamino, alkoxycarbonyl amino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, acylamino or carboxyl; n4 represents an integer of 0 to 4; and Ar, R5, R6, and Rf preferably include 1 to 6 SO₃H or salts thereof in total.

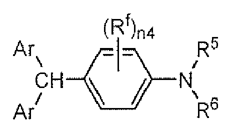

wherein, Ar, R⁵, R⁶, R^{f}, and n4 are as defined above.

It is also possible to use the following compound as an electrophoresis reagent of the present invention.

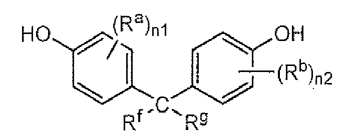

wherein, R^{a}, R^{b}, n1, and n2 are as defined above; one of R^{f} and R^{g} represents a hydrogen atom and the other represents substituted or unsubstituted aryl sulfonic acid, or R^{f} and R^{g} together represent an aromatic or heteroaromatic group having a sulfo group.
Examples of substituted or unsubstituted aryl sulfonic acids include substituted or unsubstituted benzene sulfonic acid, substituted or unsubstituted naphthalene sulfonic acid, substituted or unsubstituted anthracene sulfonic acid, substituted or unsubstituted phenanthrene sulfonic acid, substituted or unsubstituted fluorene sulfonic acid, substituted or unsubstituted indane sulfonic acid, substituted or unsubstituted indene sulfonic acid, substituted or unsubstituted tetralin sulfonic acid, and substituted or unsubstituted acenaphthene sulfonic acid. Examples of substituted or unsubstituted aromatic groups having a sulfo group include substituted or unsubstituted indene, indane, tetralin, or fluorene having a sulfo group. Examples of substituted or unsubstituted heteroaromatic group having a sulfo group include substituted or unsubstituted benzofuran, benzopyran, xanthene, chromene, isobenzofuran, indoline, or isoindoline having a sulfo group. The chemical formulae of these compounds are shown below.

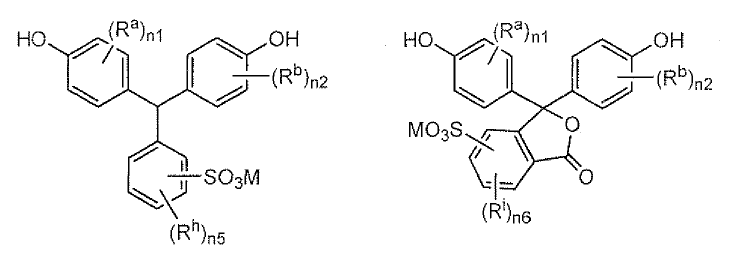

wherein, R^{a}, R^{b}, n1 n2, M are as defined above; R^{h} and Rⁱ, which are the same or different, represent a sulfo group (SO₃H) or a salt thereof, a halogen atom, alkyl, alkenyl, alkynyl, alkoxy, methylenedioxy, hydroxy, trifluoromethoxy, trifluoroethoxy, trifluoromethyl, cyano, nitro, amino, monoalkylatnino, dialkylamino, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, acylamino or carboxyl; and n5 and n6 are integers of 0 to 4.

Preferable examples of an acid dye that produces the compound of the present invention through a reduction process include CBB R150, CBB G250, CBB R250, and CBB R350.

For example, the compounds represented by Formulae (Ig) and (Ih) can be obtained by reducing commercially available CBB G250 or CBB R250 using a standard method. The compound represented by Formula (Ia) can be easily obtained by synthesizing a CBB Compound (IIc) using the same synthesis method as that for CBB G250 or CBB R250, followed by reduction using a reducing agent (see Scheme 2).

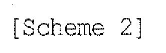

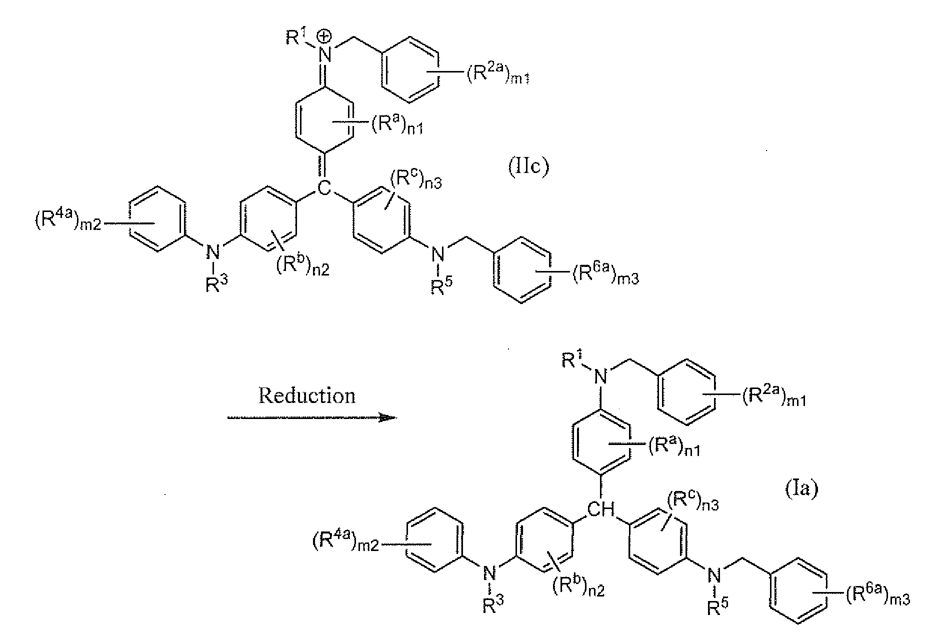

wherein, R¹, R³, R⁵, R^{a}, R^{b}, R^{c}, R^{2a}, R^{4a}, R^{6a}, n1, n2, n3, m1, m2, and m3 are as defined above.

The reduction can be performed in a solvent in the presence of a reducing agent, such as sodium cyano boron hydride, sodium boron hydride, LiAlH₄ or the like. Examples of such solvents include alcohols, such as methanol, ethanol etc. The reduction reaction advantageously proceeds by reacting 1 mol of compound of Formula (IIc) for 30 minutes to 24 hours at a temperature from 0°C to the boiling point of the solvent using 1 mol to an excessive amount of a reducing agent.

The following shows a structure of a preferable compound of the present invention.

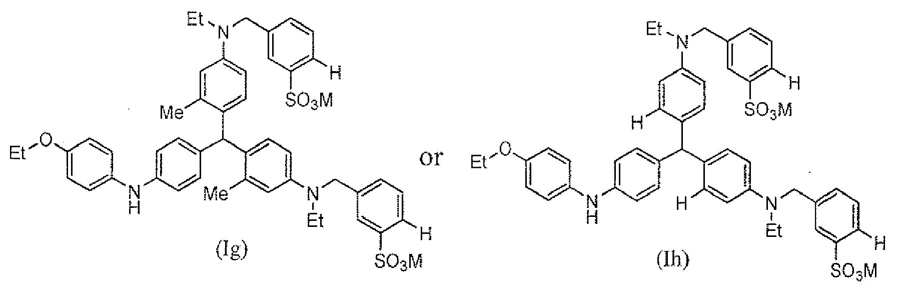

wherein M represents a hydrogen atom or a water-soluble salt.

Compound (I) of the present invention has a protein binding property. Further, Compound (I) is substantially colorless because it exhibits little absorption of visible light. With these characteristics, Compound (I) is capable of applying a negative charge to a protein having a basic isoelectric point, a membrane protein, etc., without adding visible light absorption.

Further, Compound (I) is highly soluble in water, and thus can be easily handled.

Further, unlike a surfactant having a protein transforming function such as SDS, the bond of Compound (I) to the molecular surface of the protein is relatively weak. Therefore, the higher-order structure of the protein or the association state of a multimeric protein will not be changed.

With these characteristics, Compound (I) is capable of the electrophoresis of proteins in a natural state, and is also useful as an electrophoresis reagent (reagent for Clear Native electrophoresis) that makes the gels resulting from electrophoresis transparent.

The electrophoresis reagent of the present invention may comprise at least one compound (i.e., Compound (I)), or may be an electrophoresis gel composition obtained by incorporating Compound (I) into an electrophoresis gel, or an electrophoresis buffer composition obtained by incorporating Compound (I) into an electrophoresis buffer (cathode buffer, anode buffer), a gel-producing buffer, or a sample buffer.

Examples of electrophoresis gels include polyacrylamide gels, agarose gels, and dextran gels. Particularly, the present invention is suitable for polyacrylamide gel electrophoresis that uses polyacrylamide gel as the electrophoresis gel.

The gels may be those having a fixed concentration or those having a gradient concentration.

For example, for polyacrylamide gels, gels having a gradient concentration ranging from 4-16% or 5-20%, and gels having a fixed concentration of 8% or 12% may be used. Generally, a sharper band is obtained when using gels having a gradient concentration than when using gels having a fixed concentration.

In the specification of the present invention, "%" denotes "w/v (%)" unless otherwise specified.

The electrophoresis buffer (sample buffer) may be similar to those used for known Blue-Native electrophoresis. However, generally, a buffer with a pH value of about 6 to 8, and a salt concentration of 0.3 M or less is suitable. For example, a buffer comprising 100 mM imidazole pH 7.0, 100 mM NaCl, 1% dodecylmaltoside, 20% glycerol, and 1% Compound (I), or a buffer comprising 20 mM Hepes pH 7.5, 150 mM NaCl, 0.1% dodecylmaltoside, 20% glycerol, and 0.1% Compound (I) may be used. The buffers used for known Blue-Native electrophoresis are also readily useful as an electrophoresis buffer (cathode buffer, anode buffer), and as a gel-producing buffer. For example, the cathode buffer may comprise 50 mM Tricine, 7.5 mM imidazole (pH value = 7.0), and 0.02% Compound (I). The anode buffer may comprise 25 mM imidazole (pH value = 7.0).

The reagent of the present invention may contain other components according to the target purposes, such as improvement in protein solubility or stabilization.

Examples of other components include solubilizers and a disulfide bond-reducing reagent. Examples of solubilizers include n-dodecyl-β-D-maltoside (DDM) and digitonin. The solubilizers serve to increase solubility without inhibiting the bond of Compound (I) and a protein. Examples of disulfide bond-reducing reagents include dithiothreitol and 2-mercaptoethanol.

The protein separating method of the present invention is a method of separating proteins by performing electrophoresis using an electrophoresis reagent or an electrophoresis composition.

The electrophoresis reagent or composition of the present invention contains Compound (I) that binds to a protein and thereby negatively charges the entire molecule without impairing the complex or the higher-order structure of the protein. Accordingly, it becomes possible to move the protein in the anode direction while maintaining its natural structure, thereby separating the protein.

The sample (specimen) of the target protein used in the protein separating method of the present invention is not limited insofar as it can be assayed. Examples of proteins include enzymes, membrane proteins, and antibodies. The protein may form a complex.

The molecular weight of the protein is not limited insofar as it can be assayed. The molecular weight is generally about 20,000 to 1,500,000 daltons, preferably about 50,000 to 1,000,000 daltons.

The sample may be a purified solution of a protein, a cell homogenate, or a solubilized cell membrane.

Further, the sample may contain one kind of protein or two or more kinds of proteins. For example, the sample may be a solution containing two or more proteins that interact with each other.

Furthermore, the sample may be subjected to a pretreatment before the electrophoresis, such as a process for removing large aggregates by way of centrifuge or filtering, or a process for creating a fluorescent protein using SYPRO Orange or the like.

Such a sample is processed into an electrophoresis sample by using the electrophoresis reagent or the composition of the present invention.

The sample can be prepared according to a known method. For example, the sample may be prepared by mixing a protein sample with a buffer composition containing Compound (I) and a sample buffer. Further, the sample may also be prepared by adding an electrophoresis reagent comprising Compound (I) to a buffer containing a solubilized protein sample, and further adding, as necessary, other components, such as glycerol.

The protein concentration differs depending on the staining or detection method used after electrophoresis, and cannot be set to a certain value; however, the concentration is generally about 0.5 to 20 µg/well, and preferably about 1 to 10 µg/well, when detection is performed by CBB staining. When detecting the fluorescence of a fluorescent protein fusion product, the concentration is generally about 0.05 to 1 µg/well, and preferably about 0.1 to 0.5 µg/well. Further, when the detection is performed by silver staining, the concentration is generally about 0.5 to 100 ng/well, and preferably about 5 to 50 ng/well. These concentrations are based on the assumption that each well contains about 20 µL of sample.

The concentration of Compound (I) in each sample is set to an appropriate value depending on the detection method. The concentration is generally about 0.02 to 0.5%, and preferably about 0.05 to 0.25%.

The electrophoresis sample thus prepared is applied to a gel using a general-purpose electrophoresis device to carry out electrophoresis to enable protein separation.

A known protein electrophoresis device may be appropriately used, such as a device comprising an electrophoresis tank, a electrophoresis glass plate, a buffer tank, a spacer, a comb, a clip, a power supply, a peristaltic pump, etc.

The electrophoresis buffer may be similar to a known buffer used for Blue-Native electrophoresis.

For example, examples of electrophoresis buffers include tris-glycine buffer, imidazole-tricine buffer, and bis-tris-tricine buffer. Electrophoresis buffers having different formulations are used for the anode and the cathode.

An example of tris-glycine buffer for a cathode buffer has the following formulation: 25 mM tris base, 192 mM glycine, pH value = 8.5. An example of tris-glycine buffer for an anode buffer has the following formulation: 25 my, tris base, 192 mM glycine, pH value = 8.5.

An example of imidazole-tricine buffer for a cathode buffer has the following formulation: 7.5 mM imidazole, 50 mM tricine, pH value = 7.0. An example of imidazole-tricine buffer for an anode buffer has the following formulation: 25 mM imidazole, pH value = 7.0.

An example of bis-tris -tricine buffer for a cathode buffer has the following formulation: 50 mum bis-tris, 50 mM tricine, pH value = 6.8. An example of tris-tricine buffer for a anode buffer has the following formulation: 50 mM bis-tris, 50 mM tricine, pH value = 6.8.

Each cathode buffer comprises Compound (I) generally in an amount of about 0.002 to 0.02%, and preferably about 0.004 to 0.01%.

The electrophoresis conditions are not particularly limited insofar as the protein retains the natural state during the electrophoresis. The conditions are appropriately set according to the type of sample and the purpose of the assay.

The temperature may also be appropriately set according to the type of protein; however, because proteins are more stable at low temperatures, the electrophoresis is usually performed at about 4°C.

Electrophoresis attracts the protein in the sample to the anode while maintaining its natural state, thus separating proteins by molecular size.

After the electrophoresis, the protein band is immobilized and made visible to allow the size, purity, interaction, etc., of the protein in its natural state to be assayed.

The detection of protein bands after the electrophoresis may be performed by ordinary CBB staining, or optionally by silver staining or western blotting. These methods make it possible to assay the protein size and association state with high accuracy even for a very small amount of sample measuring several nanograms. The Clear Native electrophoresis of the present invention features extremely high sensitivity.

Also, because the gel resulting from electrophoresis using Compound (I) is transparent, silver staining can be carried out without having to wash Compound (I).

Further, because the gel resulting from electrophoresis using Compound (I) is transparent, it is possible to detect the color or fluorescence derived from the protein immediately after electrophoresis. For example, by expressing a fusion protein of a fluorescence protein such as GFP and a protein as the study object using an appropriate expression host, and subjecting the crude extract containing a fluorescence protein chimera to electrophoresis, the expression amount and the association state can be estimated without purification. In addition, the extract may be directly used for measuring enzymatic activity that involves chromogenic reactions, such as ATP hydrolysis, peroxidase reaction, galactosidase reaction, dehydrogenase reaction or the like.

It is also possible to perform two-dimensional electrophoresis that combines the method of electrophoresis using Compound (I) of the present invention and another electrophoresis separation such as SDS-PAGE. For example, in the first dimension, electrophoresis is performed using Compound (I) of the present invention to separate the complexes in their association states. In the second dimension, electrophoresis is performed with SDS-PAGE to dissociate the complex, thereby assaying the higher-order structure of the complex and the polypeptide chain.

The electrophoresis reagent and the composition of the present invention may be provided in the form of an electrophoresis kit by combining them with various reagents or electrophoresis devices as required.

For example, the kit may comprise one or more of the following: a molecular weight marker, a coloring reagent, a blotting buffer, and a blotting membrane, in addition to the above electrophoresis reagent, the electrophoresis buffer composition, and the electrophoresis gel composition of the present invention.

Further, the kit may comprise one or more ordinary electrophoresis devices and components, i.e., an electrophoresis tank, an electrophoresis glass plate, a buffer tank, a spacer, a comb, a clip, a power supply, a peristaltic pump, etc.

The electrophoresis reagent and the composition, the protein separation method and the electrophoresis kit of the present invention may be combined with known technologies regarding protein electrophoresis, in particular, regarding Native electrophoresis, as required.

According to the present invention, the gel resulting from electrophoresis is transparent; therefore, the present invention does not require a colored reagent in the gel to be washed before subsequent detection using silver staining or western blotting.

Further, since the gel resulting from electrophoresis is transparent, it is possible to detect a color or fluorescence derived from the protein directly resulting from the electrophoresis. This enables the protein to be assayed with high accuracy even for a very small amount of protein in a crude sample. For example, by processing the target protein into a fusion protein with GFP and observing the GFP fluorescence, the association state and the molecular weight may be assayed with high accuracy immediately after electrophoresis. Moreover, since the activity of the protein is retained during the electrophoresis, it is possible to measure the enzymatic activity of the protein by a chromogenic reaction immediately after the electrophoresis.

In addition, since the cathode buffer is transparent, it is possible to easily confirm whether the protein sample is properly loaded to a sample well, thereby simplifying the handling of the sample and the operation of the electrophoresis.

As explained, the present invention provides a simple method for assaying the natural state and the enzymatic activity of the protein with high accuracy. With this advantage, the present invention greatly contributes to the research and the development of its applied technologies for important membrane proteins and supramolecular assemblies.

### Examples

The present invention is more specifically explained below in reference to Examples. The present invention is, however, not limited to these Examples.

In the Examples below, Blue Native PAGE is referred to as "BN-PAGE" and Clear Native PAGE is referred to as "CN-PAGE".

Further, although the Examples below show the results obtained by polyacrylamide electrophoresis (PAGE), the present invention is, of course, effective for electrophoresis of other proteins.

### Example 1

### 1. Production of a Compound Represented by General Formula (Ig)

Methanol (60 mL) and 2.65 g (42.3 mmol) of sodium cyanoboronhydride were added to (benzenemethanaminium, N-[4-[[4-[(4-ethoxyphenyl)amino]phenyl][4-[ethyl[(3-sulfophenyl)methyl]amino]phenyl]methylene]-2,5-cyclohexadien-1-ylidene]-N-ethyl-3-sulfo-, hydroxide, inner salt, monosodium salt) (CBB G250) 12.0 g (14.1 mmol) at 0 °C, and the mixture was stirred for 1.5 hours at room temperature.

After concentrating the reaction mixture under reduced pressure, subsequent recrystallization from a mixed solution of diethyl ether and methanol yielded 8.95 g (yield = 73%) of sodium 3,3'-(4,4'-((4-(4-ethoxyphenylamino)phenyl)methylene)bis(3-ethyl-4,1-phenylene))bis(ethylazanediyl)bis(methylene)dibenzenesulfonate(so dium3,3'-(4,4'-((4-(4-ethoxyphenylamino)phenyl)methylene)bis(3-methyl-4,1-phenylene))bis(ethylazanediyl)bis(methylene)dibenzenesulfonate (hereinafter referred to as Compound (Ig)).

The NMR analysis of Compound (Ig) is shown below. 1H NMR (270 MHz, CD3OD): δ7.65 -7.85 (m, 4H), 7.37 (brs, 4H), 7.00 (d, J = 9.2Hz, 2H) 6.70 -6.90 (m, 6H), 6.35 -6.57 (m, 6H), 5. 36 (s, 1H), 4.50 (s,4H), 3.97 (q,J = 6.6 Hz,2H), 3.43 (q,J = 6.6 Hz,4H), 2.03 (s,6H), 1.34 (t,J= 6.6 Hz,3H), 1.15 (t,J = 6.6 Hz,6H).
The above reaction is shown below as a reaction scheme.

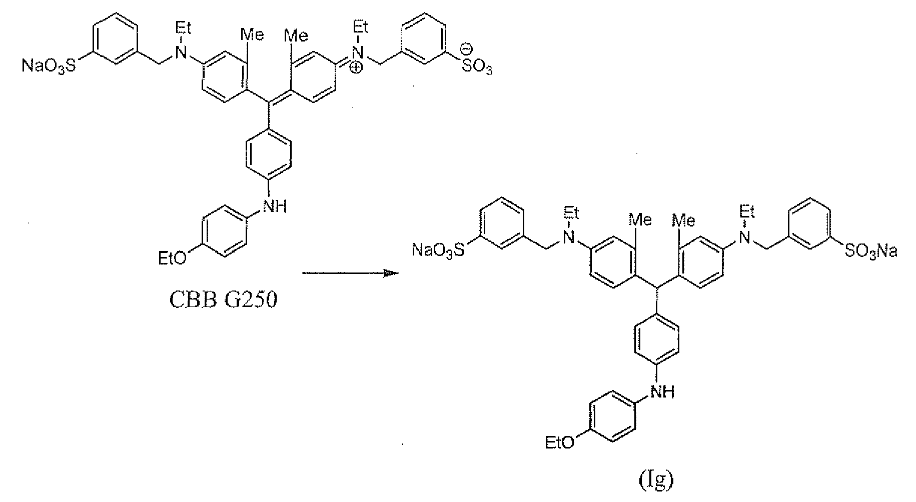

Below, Compound (Ig) thus obtained was used as a Clear Native electrophoresis reagent.

### 2. Absorbance Spectrum of Compound (Ig)

Compound (Ig) and commercial CBB G250 (Nacalai Tesque, Inc.) were dissolved in water, and the visible absorption spectrum was measured using a spectrophotometer (Hitachi UV-2810).

FIG. 1 shows the results.

As shown in FIG. 1, the light absorption of Compound (Ig) at 582 nm (the absorption maximum of CBB G250) was decreased to 1/1000th that of CBB G250. Further, no color was observed in a 1 mg/ml aqueous solution of Compound (Ig) according to visual judgment.

### 3. CN-PAGE Using Compound (Ig)

Native PAGE using Compound (Ig) and CBB G250 were performed for a gel filtration molecular weight marker (GE Healthcare) and three kinds of membrane proteins, and each pattern was compared.

The electrophoresis conditions are shown below.
(1) Electrophoresis system
   Xcell SureLock mini cell and Power Ease 500 power supply
   (Invitrogen Corporation)
(2) Cathode solution
   BN-PAGE
   50 mM tricine, 7.5 mM imidazole, and 0.02% (W/V) CBB G250 (pH value = 7.0)
   CN-PAGE
   50 mM tricine, 7.5 mM imidazole, and 0.02% (W/V) Compound (Ig)
   (pH value = 7.0)
(3) Anode solution
   25 mM imidazole (pH value = 7.0)
The sample for BN-PAGE was prepared by dissolving a protein sample (20 µg) in a solution containing 50 mM imidazole, 50 mM NaCl, 0.5% dodecylmaltoside, and 0.5% CBB G250. The sample for CN-PAGE was prepared in the same manner as described above except that Compound (Ig) was used instead of CBB G250.

Polyacrylamide gel (Native PAGE TM 4-16% bis-tris gel, 1.0 mm, 10 wells, Invitrogen Corporation) was used.

Hereinafter, Native electrophoresis using CBB G250 is also referred to as Blue Native PAGE (or BN-PAGE), and Native electrophoresis using Compound (Ig) is also referred to as Clear Native PAGE (or CN-PAGE).

The sample was loaded to each well, and electrophoresis was performed by applying a voltage of 150 V for one hour, and then 250 V for another hour. After the electrophoresis, each sample was destained using a 30% (V/V) methanol-10% (V/V) acetic acid aqueous solution for BN-PAGE, and using Imperial Stain (Pierce) for CN-PAGE.

Soluble proteins used as protein samples included Thyroglobulin (molecular weight = 669 kDa), Ferritin (molecular weight = 440 kDa), Aldolase (molecular weight = 158 kDa), Conalbumin (molecular weight = 75 kDa), Ovalbumin (molecular weight = 43 kDa), Carbonic Anhydrase (molecular weight = 29 kDa), and RNase A (molecular weight = 13.7 kDa).

Further, as the membrane protein, nitric oxide reduction enzyme (NOR, molecular weight = 68.5 kDa), Band 3 (molecular weight = 55 kDa (110 kDa)), and adenosine receptor A2a (A2a, molecular weight 33 kDa) were used.

FIG. 2 shows the results.

As shown in FIG. 2, substantially the same pattern was observed in CN-PAGE as that in BN-PAGE for both water-soluble proteins and membrane proteins.

The result showed that, like CBB G250, Compound (Ig) binds to a protein; therefore, Compound (Ig) can be used for electrophoresis.

Further, in BN-PAGE, the cathode buffer had a deep blue color, which made it difficult to confirm the proper loading of the sample when the sample was loaded to each well, or to detect any leakage of the sample from the well; however, in CN-PAGE, because the cathode buffer was transparent, there was no such difficulty, and application of the sample to the gel was very easy.

### 4. In-Gel Fluorescence Detection

A fusion protein of a fluorescence protein and a membrane protein was prepared, and CN-PAGE using Compound (Ig) was performed, followed by fluorescence detection.

EGFP was used as a fluorescence protein. EGFP was expressed using an EGFP-expression plasmid and a budding yeast as a host, followed by purification using Ni-NTA super flow (Qiagen).

Further, a fusion protein of adenosine receptor A2a and EGFP was used as a fusion protein (hereinafter referred to as a membrane protein-EGFP fusion protein). The membrane protein-EGFP fusion protein was expressed using a plasmid DNA encoding a A2a-EGFP fusion protein and a budding yeast as a host. The collected yeast cells were disrupted with glass beads, and the membrane fraction was separated by ultracentrifugation.

The membrane fraction of budding yeast thus prepared containing EGFP (75 ng) and the membrane protein-EGFP fusion protein (about 50 ng) was solubilized by 1% dodecylmaltoside, followed by electrophoresis using Compound (Ig). The image of the gel directly resulting from electrophoresis was captured using LAS-1000 plus (Fujifilm) under 470 nm LED excitation light using a 515-nm fluorescence low-pass filter. The same electrophoresis conditions as in Item 3 above were used. FIG. 3 shows the results.

As shown in FIG. 3, after CBB staining, the purified EGFP was faintly detected; however, EGFP was detected very clearly by photofluorography. For the membrane protein-GFP fusion protein, band identification by CBB staining failed due to a large amount of other contaminating proteins; however, the molecular weight and the association state were clearly detected by photofluorography. Generally, a complicated analytical practice including western blotting, purification, gel filtration and the like is required to find the protein expression amount or the association state thereof; however, the above results showed that the subject method enables very simple detection of such data in a short time.

### 5. In-Gel Enzymatic Activity Detection

BN-PAGE and CN-PAGE were performed using β-galactosidase derived from Escherichia Coli, and the enzymatic activity in the gel was detected.

The same conditions as in the above Item 3 were used in electrophoresis. After the electrophoresis, the gel was stained by incubation for 20 minutes with 0.01% nitro blue tetrazolium, 0.05% X-gal in PBS, pH value = 7.0.

FIG. 4 shows the results. The left side of FIG. 4 shows the gel after BN-PAGE. The right side of FIG. 4 shows the gel after CN-PAGE. The application amount of β-galactosidase was the same for both cases. The amounts were 10 µg, 5 µg, 2.5 µg, 1.25 µg, 1 µg, 0.5 µg, 0.25 µg, and 0.125 µg, from the right lane to the left.

As shown in FIG. 4, the enzymatic activity was retained in both cases. However, the colored band is not clearly visible in BN-PAGE due to the remaining CBB G250; however, the band is clearly visible in CN-PAGB.

### 6. Silver Staining

CN-PAGE was performed using a membrane protein and a water-soluble protein as protein samples, followed by silver staining.

A nitric oxide reduction enzyme derived from Pseudomonas aeruginosa (hereinafter referred to as "NOR", molecular weight = 68.5 kDa) was used as the membrane protein. NOR was prepared by culturing Pseudomonas aeruginosa, subjecting the cultured cells to ultrasonic fragmentation to collect the membrane fraction, and solubilizing the fraction with a surfactant, followed by purification by chromatography.

Bovine serum albumin (BSA, molecular weight 66 kDa, Sigma-Aldrich) was used as a water-soluble protein.

The same electrophoresis conditions as those in the above Item 3 were used, except that the amounts of dodecylmaltoside and Compound (Ig) in the sample buffer were 0.02% and 0.05%, respectively, and the amount of Compound (Ig) in the cathode buffer was 0.002%. The Silver Staining Kit II (Wako Pure Chemical Ind. Ltd.) was used for silver staining after electrophoresis.

FIG. 5 shows the results. FIG. 5 (A) shows the results of the NOR sample. FIG. 5 (B) shows the results of the BSA sample. "M" in the horizontal axis denotes the marker. The amounts of protein used for electrophoresis were 1,000 ng, 500 ng, 100 ng, 50 ng, 10 ng, 5 ng, 1 ng, 0.5 ng, 0.1 ng from left to right starting from the lane next to the marker.

As shown in FIGS. 5 (A) and (B), band detection was possible up to about 0.5 ng of protein for both cases. This showed that, by adopting CN-PAGE, silver staining immediately after electrophoresis becomes possible, thereby enabling analysis with a small amount of protein sample.

Further, as shown in FIG. 5 (B), not only a BSA monomer band but also bands having some association states derived from interaction between BSA molecules were detected.

The above results show that Clear Native electrophoresis using Compound (I) is superior to conventional Blue Native electrophoresis in terms of handling ease and detection sensitivity. Moreover, the results also reveal that, since the above method performs highly sensitive detection using fluorescent materials or silver staining, it enables clear detection of the size of the protein and the expression amount of a very small amount of protein sample; it also makes it possible to assay interactions between the proteins or the like.

## Claims

1. An electrophoresis reagent comprising at least one substantially colorless compound that binds to a protein in a neutral electrophoresis buffer and thereby forms a complex in which entire molecules are negatively charged, the compound being represented by Formula (I) below: 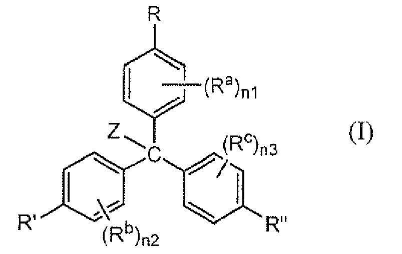 wherein, R represents a hydrogen atom, SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, carboxyl or NR¹R²; R' represents a hydrogen atom, SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, carboxyl or NR³R⁴; R" represents a hydrogen atom, SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, carboxyl or NR⁵R⁶; R¹, R², R³, R⁴, R⁵, and R⁶, which are the same or different, represent a hydrogen atom, alkyl, cycloalkyl, substituted or unsubstituted aryl or substituted or unsubstituted aralkyl; R^{a}, R^{b}, and R^{c}, which are the same or different, represent SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, amino, monoalkylamino, dialkylamino, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, acylamino or carboxyl; n1, n² and n³, which are the same or different, represent an integer of 0 to 4; and Z represents a hydrogen atom, a halogen atom, OH, alkoxy, alkylcarbonyloxy, arylcarbonyloxy, aryloxy, SH, alkylthio, alkyl, amino (NH₂), monoalkylamino, dialkylamino or acylamino, provided that the compound of Formula (I) comprises 1, 2, 3, 4, 5 or 6 SO₃H or salts thereof.

2. The electrophoresis reagent according to claim 1, comprising at least one compound represented by General Formula (Ia) below, 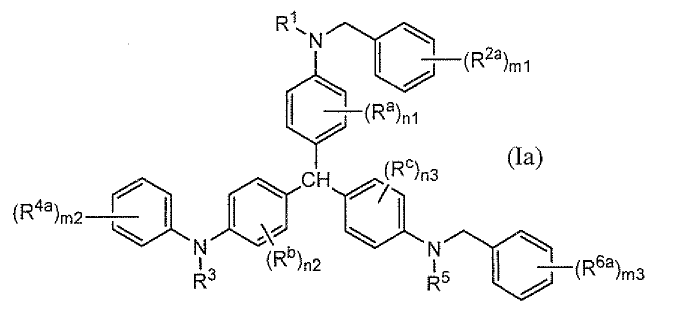 wherein, R¹, R³, and R⁵, which are the same or different, represent a hydrogen atom, alkyl, cycloalkyl, substituted or unsubstituted aryl or substituted or unsubstituted aralkyl; R^{a}, R^{b}, R^{c}, R^{2a}, R^{4a} and R^{6a}, which are the same or different, represent SO₃H or a salt thereof, a halogen atom, alkyl, alkenyl, alkynyl, alkoxy, methylenedioxy, hydroxy, trifluoromethoxy, trifluoroethoxy, trifluoromethyl, cyano, nitro, amino, monoalkylamino, dialkylamino, alkoxycarbonylamino, carbamoyl, mono- or di-alkylcarbamoyl, sulfamoyl, mono- or di-alkylsulfamoyl, alkylsulfonylamino, alkoxycarbonyl, alkylcarbonyloxy, arylcarbonyloxy, aryloxy, acylamino or carboxyl; n¹, n² and n³, which are the same or different, represent an integer of 0 to 4; and m¹, m² and m³, which are the same or different, represent an integer of 0 to 5, provided that the compound of Formula (Ia) comprises 1, 2, 3, 4, 5 or 6 SO₃H or salts thereof, and 1 to 6 of R¹, R³, R⁵, 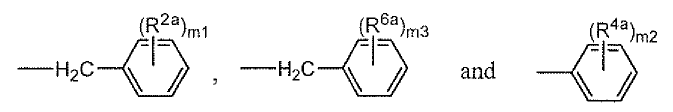 represent aryl or aralkyl having at least one SO₃H or salt(s) thereof.

3. The electrophoresis reagent according to claim 1 or 2, comprising at least one compound represented by General Formula (Ib) below, 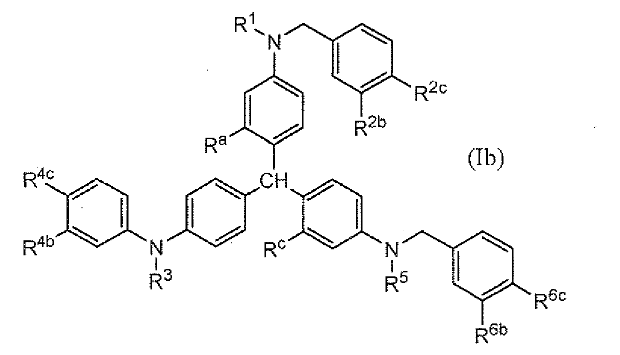 wherein, R¹, R³ and R⁵, which are the same or different, represent a hydrogen atom, C₁₋₄alkyl, substituted or unsubstituted phenyl or substituted or unsubstituted benzyl; R^{a} and R^{c}, which are the same or different, represent a hydrogen atom or methyl; one of R^{2b} and R^{2c} is a hydrogen atom while the other is SO₂NR⁹R¹⁰, SO₃H or a salt thereof; one of R^{4b} and R^{4c} is a hydrogen atom while the other is alkoxy; one of R^{6b} and R^{6c} is a hydrogen atom while the other is SO₂NR⁹R¹⁰, SO₃H or a salt thereof; R⁹ and R¹⁰ represent hydrogen or alkyl, provided that one or two of R^{2b}, R^{2c}, R^{6b} and R^{6c} represent SO₃H or salts thereof.

4. The electrophoresis reagent according to any one of claims 1 to 3, comprising at least one compound represented by General Formula (Ic) below, 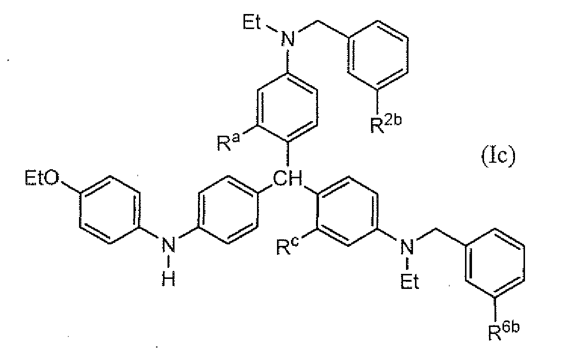 wherein, R^{a} and R^{c}, which are the same or different, represent a hydrogen atom or methyl; and R^{2b} and R^{6b} represent SO₃H or a salt thereof.

5. An electrophoresis gel composition containing an electrophoresis gel and the electrophoresis reagent according to any one of claims 1 to 4.

6. An electrophoresis buffer composition containing an electrophoresis buffer and the electrophoresis reagent according to any one of claims 1 to 4.

7. A protein separation method comprising adding the electrophoresis reagent according to any one of claims 1 to 4 to a protein sample and/or an electrophoresis buffer; and performing Clear Native electrophoresis of a protein.

8. A kit for protein electrophoresis, comprising at least one member selected from the group consisting of: the electrophoresis reagent according to any one of claims 1 to 4; the electrophoresis gel composition according to claim 5; and the electrophoresis buffer according to claim 6.

9. A compound represented by one of Formulae (Ig) and (Ih) below, 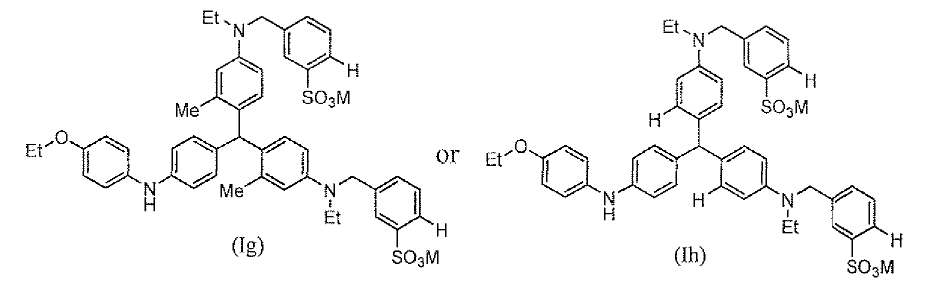 wherein M represents a hydrogen atom, a metal that forms a water-soluble salt, or N(R⁸)₄ (R⁸, which is the same or different, represents a hydrogen atom, alkyl, alkoxyalkyl, hydroxyalkyl, substituted or unsubstituted aryl, or substituted or unsubstituted aralkyl).
